# EUROPEAN PATENT SPECIFICATION

(11) **EP 1 697 326 B1**
(45) Date of publication and mention of the grant of the patent: **03.08.2011**
(21) Application number: 04807807.5
(22) Date of filing: 17.12.2004
(51) Int. Cl.: C07D 215/14, A61K 31/47

(54) **CRYSTAL FORM OF QUINOLINE COMPOUND AND PROCESS FOR ITS PRODUCTION**
KRISTALLFORM EINER CHINOLINVERBINDUNG UND VERFAHREN ZU DEREN HERSTELLUNG
FORME CRISTALLINE D'UN COMPOSE DE QUINOLINE, ET METHODE DE PRODUCTION DE LA FORME CRISTALLINE

(30) Priority: 26.12.2003 JP 2003431788
(43) Date of publication of application: 06.09.2006
(73) Proprietor: Nissan Chemical Industries, Ltd., Chiyoda-ku, Tokyo 101-0054 (JP)
(72) Inventor: OHARA, Yoshio, Nissan Chemical Industries, Ltd., Funabashi-shi, Chiba 2748507 (JP); TAKADA, Yasutaka, Nissan Chemical Industries, Ltd., Funabashi-shi, Chiba 2748507 (JP); MATSUMOTO, Hiroo, Nissan Chemical Industries, Ltd., Funabashi-shi, Chiba 2748507 (JP); YOSHIDA, Akihiro, Nissan Chemical Industries, Ltd., Funabashi-shi, Chiba 2748507 (JP)
(74) Representative: Hartz, Nikolai
(86) International application number: PCT/JP2004/019451
(87) International publication number: WO 2005/063711

(56) References cited:
- EP-A- 0 520 406
- WO-A-03/016317
- WO-A-2004/072040
- WO-A1-03/064392
- WO-A2-03/064382
- SUZUKI M ET AL: "First Systematic Chiral Syntheses of Two Pairs of Enantiomers with 3,5-dihydroxyheptenoic Acid Chain, Associated with a Potent Synthetic Statin NK-104" BIOORGANIC & MEDICINAL CHEMISTRY LETTERS, OXFORD, GB, vol. 9, no. 20, 18 October 1999 (1999-10-18), pages 2977-2982, XP004180521 ISSN: 0960-894X cited in the application
- DRUGS OF THE FUTURE, vol. 23, no. 8, 1998, pages 847-859, XP009047388

## Description

### TECHNICAL FIELD

The present invention relates to a crystal form of pitavastatin calcium known by a chemical name monocalcium bis[(3*R*,5*S*,6*E*)-7-(2-cyclopropyl-4-(4-fluorophenyl)-3-quinolyl)-3,5-dihydroxy-6-heptenoate], which is useful for treatment of hyperlipemia, as a HMG-CoA reductase inhibitor, a process for its production, and a pharmaceutical composition comprising this compound and a pharmaceutically acceptable carrier.

Particularly, it relates to pitavastatin calcium in a crystal form, which is characterized by containing from 5 to 15% (W/W) of water and which is useful as a drug substance for pharmaceuticals, from the viewpoint of the stability, etc., a process for its production, and a pharmaceutical composition containing it.

### BACKGROUND ART

Pitavastatin calcium (see Patents Documents 1, 2 and 3) is commercially available as an antihyperlipemic treating agent, and as its production method, a method of optical resolution employing optically active α-methylbenzylamine has already been reported (see Patent Document 4 and Non-patent Document 1).

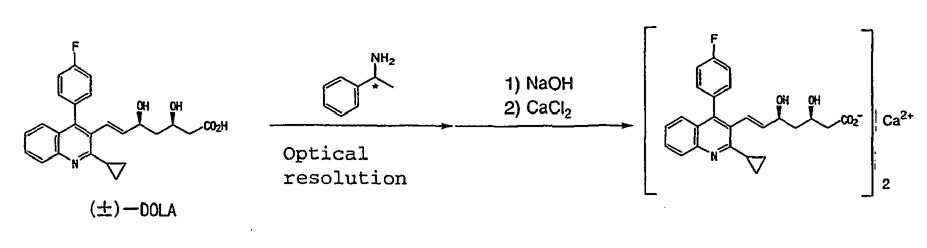

Known as methods for producing the compound of the formula (3) as the starting material, are:
column chromatographic separation employing an optical isomer separation column (see Patent Document 5),
•asymmetric synthesis (see Patent Documents 6 and 7),
•method of subjecting to chemical syn reduction a compound of the formula (4) which may be produced by using chiral synthon (see Patent Document 8),
•method of subjecting to a biological syn reduction a compound of the formula (4) (see Patent Document 9), and
•optical resolution employing an enzyme (see Patent Document 10). 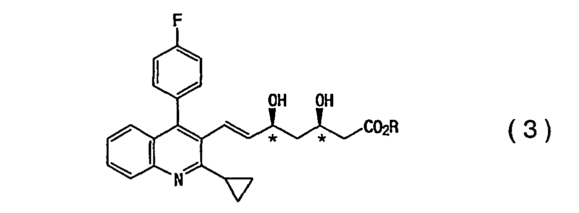 wherein R is a C₁₋₄ alkyl group. 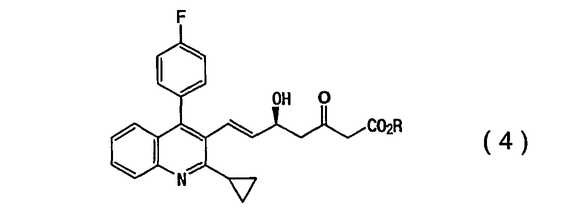
wherein R is a C₁₋₄ alkyl group.
Patent Document 1: JP-A-1-279866
Patent Document 2: EP304063A
Patent Document 3: U.S. Patent No. 5,011,930
Patent Document 4: JP-A-5-148237
Patent Document 5: WO95/23125
Patent Document 6: WO03/042180
Patent Document 7: JP-A-8-092217
Patent Document 8: JP-A-8-127585
Patent Document 9: JP-A-2002-300897
Patent Document 10: JP-A-13-352996
Non-patent Document 1: Bioorganic & Medicinal Chemistry Letters, 9 (1999), p. 2977

### DISCLOSURE OF THE INVENTION

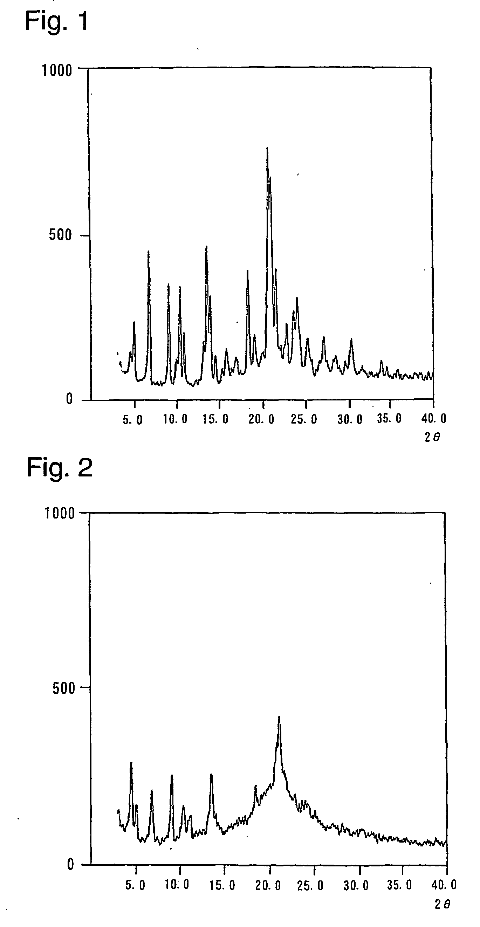

A drug substance for pharmaceuticals is desired to have high quality and a stable crystal form from the viewpoint of the storage and is further required to be durable for the production in a large scale. However, in the conventional method for producing pitavastatin calcium, there has been no disclosure relating to the water content or the crystal form. It has been found that if pitavastatin calcium (crystal form A) is subjected to drying in a usual manner, the crystallinity will decrease to a state close to an amorphous state as shown in Fig. 2 when the water content becomes to be at most 4%, even with one which shows the powder X-ray diffraction as shown in Fig. 1 prior to the drying. Further, it has been found that the pitavastatin calcium which has become amorphous, has very poor stability during the storage, as shown in Table 1.

**TABLE 1: Stability data of drug substance (influence of the water content)**

| Storage conditions | Measured item | Storage period | | | |
|---|---|---|---|---|---|
| | | Initial stage | 30 Days | 60 Days | 90 Days |
| 40°C air tight | Water content (%) | 7.89 | 7.85 | 7.88 | 7.81 |
| | Analogous substance (%) | 0.179 | 0.208 | 0.189 | 0.211 |
| | Pitavastatin calcium (%) | 99.38 | 99.42 | 99.79 | 99.64 |
| Open air | Water content (%) | 7.89 | 2.45 | 1.99 | 1.77 |
| | Analogous substance (%) | 0.179 | 0.742 | 1.347 | 2.099 |
| | Pitavastatin calcium (%) | 99.38 | 99.26 | 97.19 | 96.49 |

It is an object of the present invention to provide a crystalline drug substance of pitavastatin calcium which is stable even if it is not stored under a special storage condition and further to make industrial mass production possible.

The present inventors have conducted an extensive study on the interrelation between the moisture and the stability of the drug substance and as a result, have found that the stability of pitavastatin calcium can be remarkably improved by controlling the water content in the drug substance within a specific range. Further, it has been found that there are three types of crystal forms having the same water content, and among them, crystal (crystal form A) characterized by the powder X-ray diffraction measured by using CuKα rays, is most preferred as a drug substance for pharmaceuticals. The present invention has been accomplished on the basis of these discoveries.

Namely, the present invention provides:
1. Crystal (crystal form A) of a compound of the formula (1) : 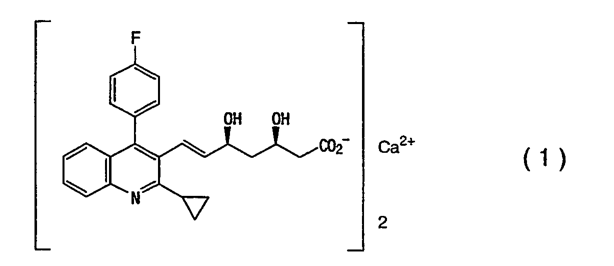 which contains from 5 to 15% of water and which shows, in its X-ray powder diffraction as measured by using CuKα radiation, a peak having a relative intensity of more than 25% at a diffraction angle (2θ) of 30.16°.
2. A process for producing the crystal (crystal form A) as defined in Item 1, which comprises adding a calcium compound to a compound of the formula (2): 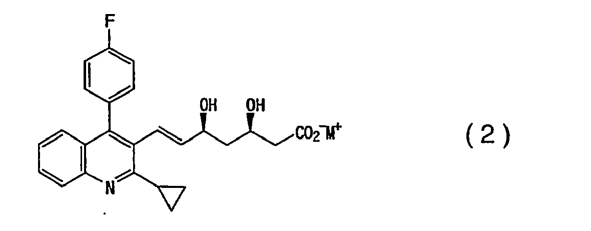 wherein M⁺ represents an alkali metal ion, dissolved in water or in a C₁₋₄ alcohol containing at least 60% of water.
3. A method for producing a drug substance of the crystal (crystal form A) as defined in Item 1, which comprises adjusting the water content to a level of from 5 to 15%.
4. A pharmaceutical composition which contains the crystal (crystal form A) as defined in Item 1.

The two types of crystal forms other than crystal form A are represented by crystal forms B and C, but neither of them shows peaks at diffraction angles 10.40°, 13.20° and 30.16° characteristic to crystal form A, thus indicating that they are crystal polymorphs. It was apparent that they are poor in filterability, require strict drying conditions (likely to undergo a change in the crystal form during the drying), are likely to include an inorganic substance such as NaCl, and are not necessarily able to maintain the reproducibility in the control of the crystal form. Thus, they have many drawbacks from the viewpoint of the industrial production method, and crystal form A is the best as a drug substance for pharmaceuticals.

### BRIEF DESCRIPTION OF THE DRAWINGS

Fig. 1 is a powder X-ray diffraction pattern of crystal form A wherein the water content is 8.78%.
Fig. 2 is a powder X-ray diffraction pattern, when the crystals used in Fig. 1 are dried to bring the water content to be 3.76%.

### BEST MODE FOR CARRYING OUT THE INVENTION

Now, the present invention will be described in detail.

Pitavastatin calcium having crystal form A is characterized by its powder X-ray diffraction pattern.

| Diffraction angle (2θ) (°) | d-lattice spacing | Relative intensity (>25%) |
|---|---|---|
| 4.96 | 17.7999 | 35.9 |
| 6.72 | 13.1423 | 55.1 |
| 9.08 | 9.7314 | 33.3 |
| 10.40 | 8.4991 | 34.8 |
| 10.88 | 8.1248 | 27.3 |
| 13.20 | 6.7020 | 27.8 |
| 13.60 | 6.5053 | 48.8 |
| 13.96 | 6.3387 | 60.0 |
| 18.32 | 4.8386 | 56.7 |
| 20.68 | 4.2915 | 100.0 |
| 21.52 | 4.1259 | 57.4 |
| 23.64 | 3.7604 | 41.3 |
| 24.12 | 3.6866 | 45.0 |
| 27.00 | 3.2996 | 28.5 |
| 30.16 | 2.9607 | 30.6 |

Apparatus:
Powder X-ray diffraction measuring apparatus: MXLabo (manufactured by MacScience)
Ray source: Cu, wavelength: 1.54056A, Goniometer: Vertical Goniometer
Monochrometer: Used, Auxiliary means: Nil, X-ray tube voltage: 50.0 Kv, Tube current: 30.0 mA

Measuring method:
Prior to the measurement, X-ray tube alignment is tested by using silicon (standard substance).

About 100 mg of a sample is put on a glass plate for the sample and flattened, followed by measurement under the following conditions.
Range of data: from 3.0400 to 40.0000 deg, Number of data points: 925
Scanning axis: 2θ/θ, θ axis angle: No setting Sampling interval: 0.0400 deg,
Scanning speed: 4.800 deg/min

The present invention also provides a production process to control pitavastatin calcium to have crystal form A.

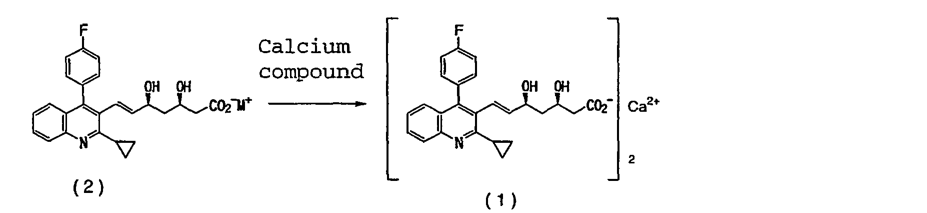

The starting material is an alkali metal salt of pitavastatin shown by the formula (2), and the alkali metal may, for example, be lithium, sodium or potassium, preferably sodium.

As the calcium compound, calcium chloride or calcium acetate may, for example, be preferred, and its amount is within a range of from 0.3 to 3 mols, preferably from 0.5 to 2 mols, per mol of the compound of the formula (2).

The alkali metal salt of pitavastatin of the formula (2) may not necessarily be isolated. For example, the Ca salt may be produced as continued from the reaction of hydrolyzing e.g. a compound of the formula (3).

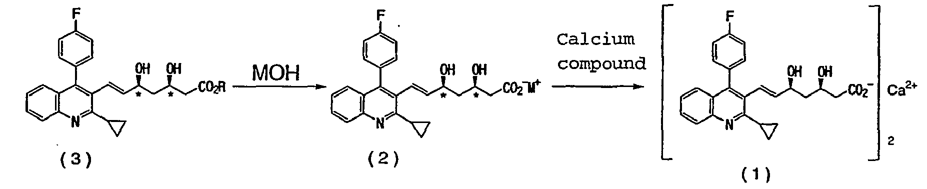

As a solvent to be used, water or a C₁₋₄ alcohol containing at least 60% of water, is preferred. The C₁₋₄ alcohol may, for example, be methyl alcohol, ethyl alcohol, n-propyl alcohol, isopropyl alcohol, n-butyl alcohol, isobutyl alcohol, sec-butyl alcohol or tert-butyl alcohol.

The amount of the solvent to be used, is usually within a range of from 3 to 100 times by mass, preferably within a range of from 5 to 30 times by mass, to the amount of the compound of the formula (2).

The crystallization temperature is not particularly limited, but it is usually within a range of from -10 to 70°C, preferably within a range of from -5 to 40°C, more preferably within a range of from 0 to 20°C.

The crystallization time is not particularly limited, but a crystallization time of from about 30 minutes to 15 hours, is usually sufficient.

As a method for crystallization, a method of carrying out the crystallization in a standing still state, or a method of carrying out the crystallization with stirring, may, for example, be mentioned. However, it is preferred to carry out the crystallization with stirring.

Further, seed crystals of crystal form A may be used as the case requires.

Precipitated crystals will then be filtered and dried. In the present invention, it is very important to adjust the water content. The drying temperature is not particularly limited, but is preferably within a range of from 15 to 40°C.

The water content is adjusted so that it will finally be within a range of from 5 to 15% (W/W), preferably within a range of from 7 to 15% (W/W), more preferably within a range of from 7 to 13% (W/W), most preferably within a range of from 9 to 13% (W/W).

The obtained pitavastatin calcium will be pulverized and then used as a drug substance for pharmaceuticals.

Administration of the compound of the present invention may, for example, be parenteral administration in the form of an injection drug (subcutaneous, intravenous, intramuscular or intraperitoneal injection), an ointment, a suppository, an aerosol or the like, or oral administration in the form of tablets, capsules, granules, pills, a syrup drug, a liquid drug, an emulsion drug or a suspension drug.

A pharmaceutical or veterinary medicine composition containing the compound of the present invention, contains from about 0.001 to 30%, preferably from about 0.01 to 10% of the compound of the present invention, based on the weight of the total composition.

In addition to the compound of the present invention or the composition containing such a compound, other pharmaceutically or veterinary medicinary active compound may be incorporated.

The clinical dosage of the compound of the present invention may vary depending upon e.g. the age, the body weight, the sensitivity of the patient or the degree of symptom. However, the effective dosage is usually at a level of from 0.003 to 100 mg, preferably from 0.01 to 10 mg, per day for an adult. However, if necessary, a dosage outside this range may be employed.

The compound of the present invention may be formulated for administration in accordance with a common method for preparation of medicines. Namely, tablets, capsules, granules or pills for oral administration may be formulated by using, for example, an excipient, such as sucrose, lactose, glucose, starch or mannitol; a binder, such as hydroxypropyl cellulose, syrup, gum arabic, gelatin, sorbitol, tragacanth, methyl cellulose or polyvinylpyrrolidone; a disintegrant, such as starch, carboxymethyl cellulose or its calcium salt, fine crystal cellulose, or polyethylene glycol; a lubricant, such as talc, magnesium or calcium stearate, or silica; a lubricating agent, such as sodium laurate or glycerol.

An injection drug, a liquid drug, an emulsion drug, a suspension drug, a syrup drug and an aerosol drug may be prepared by using, for example, a solvent for the active ingredient, such as water, ethyl alcohol, isopropyl alcohol, propylene glycol, 1,3-butylene glycol or polyethylene glycol; a surfactant, such as sorbitan fatty acid ester, polyoxyethylene sorbitan fatty acid ester, polyoxyethylene fatty acid ester, polyoxyethylene ether of hydrogenated castor oil, or lecithin; a suspending agent, such as carboxymethyl sodium salt, or a cellulose derivative such as methyl cellulose, tragacanth, a natural rubber such as gum arabic; a preservative, such as a p-hydroxybenzoate, benzalkonium chloride or a sorbic acid salt.

For an ointment which is a percutaneous absorption type formulation, white petrolatum, liquid paraffin, a higher alcohol, macrogol ointment, hydrophilic ointment or an aqueous gel base material may, for example, be used.

A suppository may be prepared by using e.g. cacao butter, polyethylene glycol, lanolin, fatty acid triglyceride, coconut oil or polysorbate.

Now, the present invention will be described in further detail with reference to Example. However, it should be understood that the present invention is by no means restricted to such a specific Example.

The compound (5) used in the Example was prepared in accordance with the method disclosed in WO95/23125.

### EXAMPLE 1

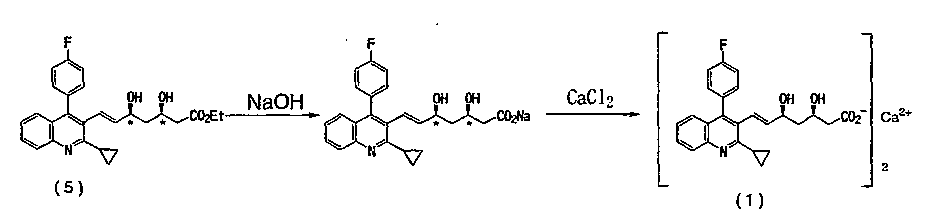

2.71 kg (6.03 mol) of the compound (5) was dissolved in 50 kg of ethanol with stirring, and after confirming the solution to be a uniform solution, 58.5 kg of water was added. After cooling it to from -3 to 3°C, 3.37 liters of a 2 mol/liter sodium hydroxide aqueous solution was dropwise added thereto, followed by stirring at the same temperature for 3 hours to complete the hydrolytic reaction. In order to introduce the entire amount of the sodium hydroxide aqueous solution to the reaction system, 4.70 kg of water was used.

The reaction mixture was distilled under reduced pressure to remove the solvent, and after removing 52.2 kg of ethanol/water, the internal temperature was adjusted to from 10 to 20°C. Into the obtained concentrated solution, a separately prepared calcium chloride aqueous solution (95% CaCl₂ 775 g/water 39.3 kg, 6.63 mol) was dropwise added over a period of 2 hours. In order to introduce the entire amount of the calcium chloride aqueous solution into the reaction system, 4.70 kg of water was used. After completion of the dropwise addition, stirring at the same temperature was continued for 12 hours, whereupon precipitated crystals were collected by filtration. The crystals were washed with 72.3 kg of water and then dried under reduced pressure in a drier at 40°C while paying an attention to the product temperature until the water content became 10%, to obtain 2.80 kg (yield: 95%) of pitavastatin calcium as white crystals.

The powder X-ray diffraction was measured to confirm the crystals to be crystal form A.

### INDUSTRIAL APPLICABILITY

According to the present invention, an industrial method for producing a crystalline drug substance of pitavastatin calcium excellent in stability, has been established.

## Claims

1. A process for producing a crystalline drug substance containing a crystal of crystal form A of a compound of the following formula (1): 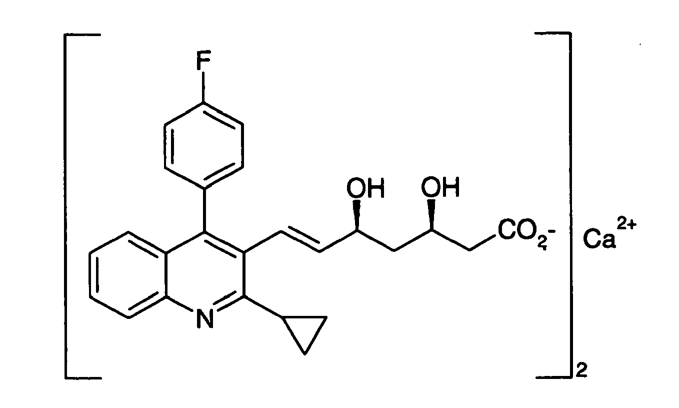 which contains from 5 to 15% of water, said process comprises
(i) hydrolyzing a compound of the formula (3) 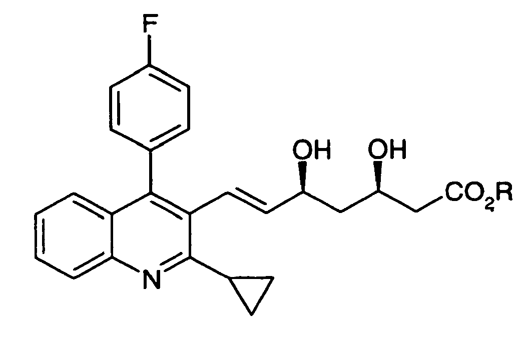 wherein R is an ethyl group, for preparing a compound of the following formula (2), wherein M represents an alkali metal ion, 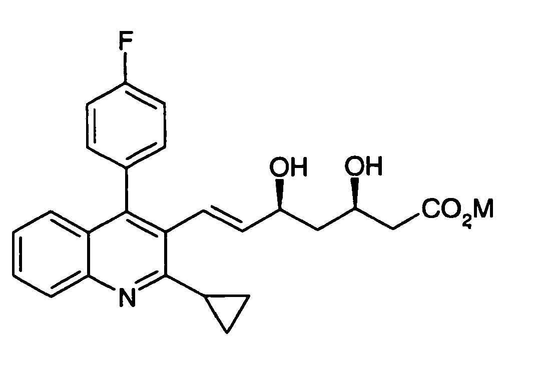
(ii) adding a calcium compound to the compound of the formula (2) dissolved in water or in C₁₋₄ alcohol containing at least 60% of water, and
(iii) adjusting the water content to a level of from 5 to15%.

2. The process according to claim 1, wherein the alkali metal is lithium, sodium or potassium.

3. The process according to any one of claims 1 or 2, wherein the calcium compound is calcium chloride or calcium acetate which is used in an amount within a range of from 0.3 to 3 mols per mol of the compound of the formula (2).

4. The process according to any one of claims 1 or 2, wherein the alkali metal salt of the formula (2) is not isolated.

5. The process according to any one of claims 1 to 4, wherein the C₁₋₄ alcohol is selected from methyl alcohol, ethyl alcohol, n-propyl alcohol, isopropyl alcohol, n-butyl alcohol, isobutyl alcohol, sec-butyl alcohol or tert- butyl alcohol.

6. The process according to any one of claims 1 to 5, wherein the crystallization temperature is within a range of from -10 to 70 °C.

7. The process according to any one of claims 1 to 6, wherein the crystallization time is from 30 minutes to 15 hours.

8. The process according to any one of claims 1 to 7, wherein the crystallization is carried out in a standing still state, or with stirring.

9. The process according to any one of claims 1 to 8, wherein the water content is adjusted at a drying temperature within a range of from 15 to 40 °C.

10. The process according to any one of claims 1 to 9, wherein the obtained crystalline drug substance is pulverized and then used as a drug substance for pharmaceuticals.

11. A pulverized drug substance obtainable by the process as defined in Claim 10.

12. A pharmaceutical composition containing a pulverized drug substance obtainable by the process as defined in Claim 10 and a pharmaceutically acceptable carrier.

13. Crystal of a compound of the following formula (1): 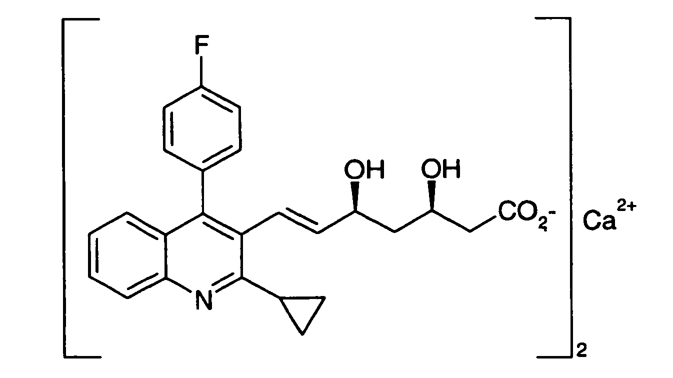 which contains from 5 to 15% of water and which shows, in its X-ray powder diffraction as measured by using CuKα radiation, a peak having a relative intensity of more than 25% at a diffraction angle (2θ) of 30.16°.

## Patentansprüche

1. Verfahren zur Herstellung einer kristallinen Arzneimittelsubstanz, enthaltend einen Kristall der Kristallform A einer Verbindung der folgenden Formel (1): 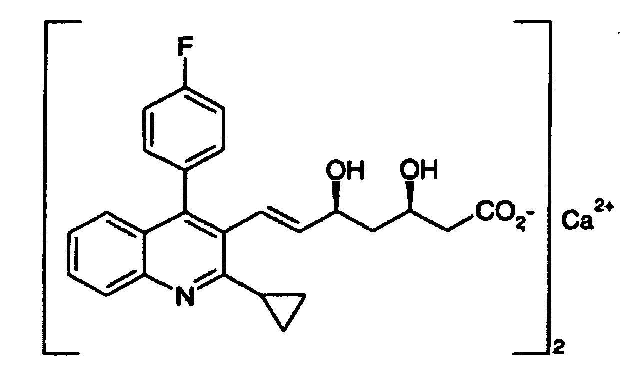 der 5 bis 15% Wasser enthält, wobei das Verfahren umfasst:
(i) Hydrolyse einer Verbindung der Formel (3) 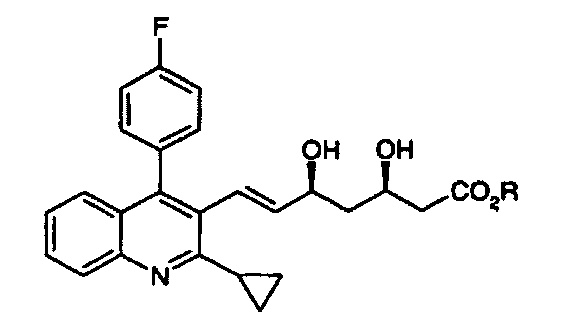 wobei R eine Ethylgruppe ist, um eine Verbindung der folgenden Formel (2) herzustellen, wobei M für ein Alkalimetallion steht, 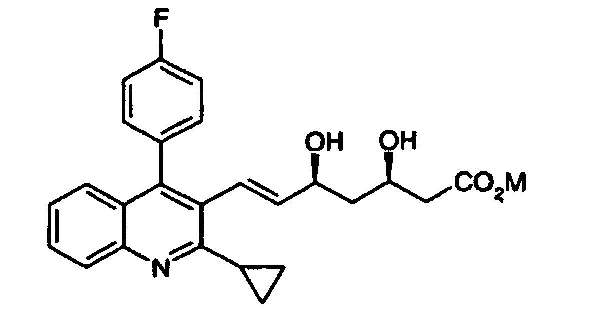
(ii) Zugabe einer Kalziumverbindung zu der Verbindung der Formel (2), die aufgelöst ist in Wasser oder einem C₁₋₄ Alkohol, der mindestens 60% Wasser enthält und
(ii) Anpassung des Wassergehalts auf ein Niveau von 5 bis 15%.

2. Das Verfahren nach Anspruch 1, wobei das Alkalimetall Lithium, Natrium oder Kalium ist.

3. Das Verfahren nach einem der Ansprüche 1 oder 2, wobei die Kalziumverbindung Kalziumchlorid oder Kalziumacetat ist, die in einer Menge verwendet wird im Bereich von 0,3 bis 3 Mol pro Mol der Verbindung der Formel (2).

4. Das Verfahren nach einem der Ansprüche 1 oder 2, wobei das Alkalimetallsalz der Formel (2) nicht isoliert wird.

5. Das Verfahren nach einem der Ansprüche 1 bis 4, wobei der C₁₋₄ Alkohol ausgewählt wird aus Methylalkohol, Ethylalkohol, n-Propylalkohol, Isopropylalkohol, n-Butylalkohol, Isobutylalkohol, sec-Butylalkohol oder tert-Butylalkohol.

6. Das Verfahren nach einem der Ansprüche 1 bis 5, wobei die Kristallisationsationstemperatur in einem Bereich von -10 bis 70 °C liegt.

7. Das Verfahren nach einem der Ansprüche 1 bis 6, wobei die Kristallisationszeit 30 Minuten bis 15 Stunden beträgt.

8. Das Verfahren nach einem der Ansprüche 1 bis 7, wobei die Kristallisation durchgeführt wird in stillstehendem Zustand, oder unter Rühren.

9. Das Verfahren nach einem der Ansprüche 1 bis 8, wobei der Wassergehalt angepasst wird bei einer Trocknungstemperatur in einem Bereich von 15 bis 40°C.

10. Das Verfahren nach einem der Ansprüche 1 bis 9, wobei die erhaltene kristalline Arzneimittelsubstanz pulverisiert wird und anschließend verwendet wird als Arzneimittelsubstanz für Pharmazeutika.

11. Pulverisierte Arzneimittelsubstanz erhältlich nach dem Verfahren wie es in Anspruch 10 definiert wird.

12. Pharmazeutische Zusammensetzung, enthaltend eine pulverisierte Arzneimittelsubstanz, erhältlich nach dem Verfahren wie es in Anspruch 10 definiert wird sowie einen pharmazeutisch unbedenklichen Träger.

13. Kristall einer Verbindung der folgenden Formel (1): 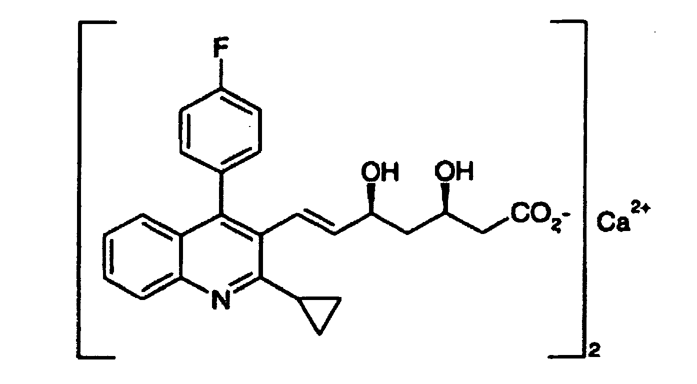 der 5 bis 15% Wasser enthält und der in der Pulver-Röntgendiffraktion, gemessen unter Verwendung von CuKα-Strahlung einen Peak aufweist mit einer relativen Intensität von mehr als 25% bei einem Beugungswinkel (2θ) von 30,16°.

## Revendications

1. Procédé pour produire une substance médicamenteuse cristalline contenant un cristal de forme cristalline A d'un composé de formule (1) suivante : 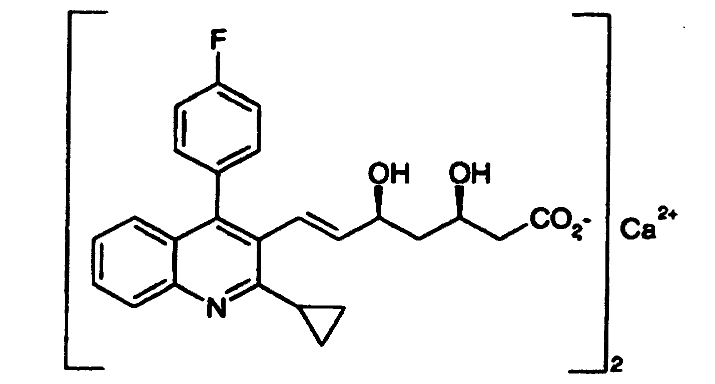 qui contient de 5 à 15 % d'eau, ledit procédé comprenant les étapes consistant à
(i) hydrolyser un composé de formule (3) 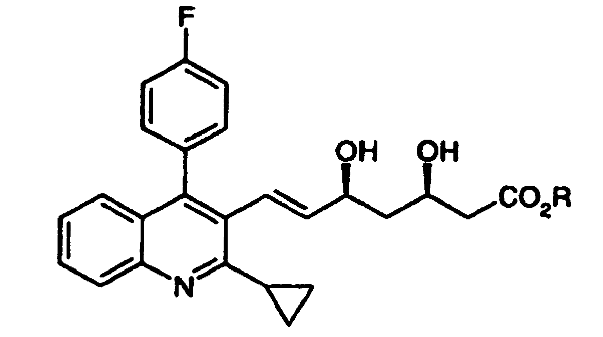 dans laquelle R est un groupe éthyle, pour préparer un composé de formule (2) suivante dans laquelle M représente un ion de métal alcalin, 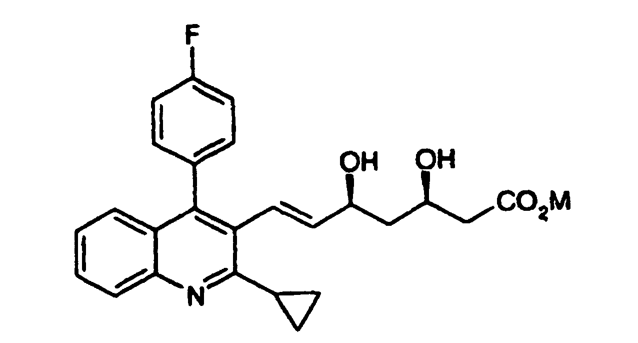
(ii) ajouter un composé du calcium au composé de formule (2) dissous dans de l'eau ou dans un alcool en C₁ à C₄ contenant au moins 60 % d'eau, et
(iii) ajuster la teneur en eau à une valeur de 5 à 15 %.

2. Procédé selon la revendication 1, dans lequel le métal alcalin est le lithium, le sodium ou le potassium.

3. Procédé selon l'une quelconque des revendications 1 et 2, dans lequel le composé du calcium est le chlorure de calcium ou l'acétate de calcium qui est utilisé en une quantité située dans la plage allant de 0,3 à 3 moles par mole du composé de formule (2).

4. Procédé selon l'une quelconque des revendications 1 et 2, dans lequel le sel de métal alcalin de formule (2) n'est pas isolé.

5. Procédé selon l'une quelconque des revendications 1 à 4, dans lequel l'alcool en C₁ à C₄ est choisi parmi l'alcool méthylique, l'alcool éthylique, l'alcool n-propylique, l'alcool isopropylique, l'alcool n-butylique, l'alcool isobutylique, l'alcool sec-butylique et l'alcool tert-butylique.

6. Procédé selon l'une quelconque des revendications 1 à 5, dans lequel la température de cristallisation est située dans la plage allant de -10 à 70°C.

7. Procédé selon l'une quelconque des revendications 1 à 6, dans lequel le temps de cristallisation est de 30 minutes à 15 heures.

8. Procédé selon l'une quelconque des revendications 1 à 7, dans lequel la cristallisation est mise en oeuvre dans un état toujours au repos, ou sous agitation.

9. Procédé selon l'une quelconque des revendications 1 à 8, dans lequel la teneur en eau est ajustée à une température de séchage située dans la plage allant de 15 à 40 °C.

10. Procédé selon l'une quelconque des revendications 1 à 9, dans lequel la substance médicamenteuse cristalline obtenue est pulvérisée et ensuite utilisée en tant que substance médicamenteuse pour produits pharmaceutiques.

11. Substance médicamenteuse pulvérisée pouvant être obtenue par le procédé tel que défini dans la revendication 10.

12. Composition pharmaceutique contenant une substance médicamenteuse pulvérisée pouvant être obtenue par le procédé tel que défini dans la revendication 10 et un véhicule pharmaceutiquement acceptable.

13. Cristal d'un composé de formule (1) suivante : 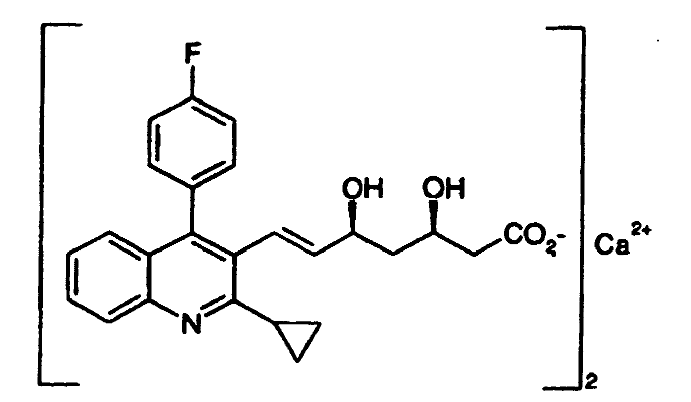 qui contient de 5 à 15 % d'eau et qui présente, dans sa diffraction des rayons X par la technique des poudres, mesurée par utilisation du rayonnement CuKα, un pic ayant une intensité relative supérieure à 25 % à un angle de diffraction (2θ) de 30,16°.
